# EUROPÄISCHE PATENTSCHRIFT

(11) **EP 0 764 468 B1**
(45) Veröffentlichungstag und Bekanntmachung des Hinweises auf die Patenterteilung: **26.07.2006**
(21) Anmeldenummer: 96114757.6
(22) Anmeldetag: 14.09.1996
(51) Int. Cl.: B01L 7/00, C12Q 1/68

(54) **System zur Temperaturwechselbehandlung von Probenflüssigkeiten**
System for cyclic thermal processing of fluid samples
Système pour le traitement thermal cyclique d'échantillons liquides

(30) Priorität: 19.09.1995 DE 19534632
(43) Veröffentlichungstag der Anmeldung: 26.03.1997
(73) Patentinhaber: Roche Diagnostics GmbH, 68305 Mannheim (DE)
(72) Erfinder: Macho, Heinz, 64658 Fürth (DE); Bienhaus, Gerhard, Dr., 82407 Wielenbach (DE)

(56) Entgegenhaltungen:
- EP-A- 0 438 883
- EP-A- 0 606 961
- EP-A- 0 642 831
- WO-A-90/08816
- WO-A-91/07486
- WO-A-93/19563
- US-A- 5 114 858
- OSTE C C: "PCR INSTRUMENTATION: WHERE DO WE STAND?" 1. Januar 1994 , POLYMERASE CHAIN REACTION, PAGE(S) 165 - 173 , MULLIS K B;FERRE F; GIBBS R A XP000575802 * das ganze Dokument *

## Beschreibung

Gegenstand der Erfindung ist ein System zur Temperaturwechselbehandlung von Nukleinsäuren, ein Verfahren zur Temperaturwechselbehandlung von Probenflüssigkeiten und ein Verfahren zum Nachweis einer Nukleinsäure in einer Probe.

Die Einstellung einer bestimmten Temperatur in einer Flüssigkeit ist bei Reaktionen, die unter Beteiligung biologisch aktiver Komponenten ablaufen, ein wichtiges Kriterium. Wenn die Temperatur nicht richtig eingestellt ist, kann es sein, daß eine bestimmte Reaktion überhaupt nicht oder nur in unerwünschtem Umfange abläuft. Dies gilt insbesondere für alle Reaktionen, bei denen Enzyme beteiligt sind. Enzyme weisen, abhängig von der Temperatur, unterschiedliche Reaktionskinetiken auf Außerdem ist die Bildung von Komplexen zwischen biologischen Bindepartnern, z. B. zueinander komplementären Nukleinsäuren, von der Temperatur abhängig. Oberhalb der Schmelztemperatur liegen die Nukleinsäuren in einzelsträngiger und unterhalb der Temperatur in doppelsträngiger Form vor. Für den Fall, daß mehrere Reaktionen hintereinander ablaufen sollen, die unterschiedliche Temperaturbedingungen erfordern, ist es erforderlich, die Temperatur des Reaktionsmediums zu ändern.

Bisher wurde dies dadurch bewirkt, daß das Gefäß, in dem sich die Reaktionsmischung befindet, zwischen Flüssigkeitsbädern unterschiedlicher Temperatur hin und her transportiert wurde. Dadurch, daß das Gefäß eine gewisse Zeit in jedes Bad eintauchte, nahm die im Gefäß befindliche Flüssigkeit die Temperatur des Temperiermediums an. Nach einer für die gewünschte Reaktion ausreichenden Zeit wurde das Gefäß mit der Flüssigkeit in ein weiteres Flüssigkeitsbad transferiert. Diese Verfahren waren naturgemäß sehr arbeitsaufwendig und schlecht automatisierbar.

In jüngerer Zeit wurden Geräte entwickelt, bei denen das Gefäß mit der zu temperierenden Flüssigkeit an einem Ort belassen, jedoch die Temperatur des Temperiermediums geändert wurde. Diese Verfahren haben jedoch den Nachteil, daß sie relativ zeitaufwendig sind, weil die Temperatur des gesamten Kühlmediums geändert werden muß. Insbesondere bei Kühlvorgängen ist dies nachteilig.

Insbesondere auf dem Gebiet der Nukleinsäurediagnostik werden Temperaturwechselbehandlungen oft eingesetzt. Beispielsweise in der Polymerase-Kettenreaktion (EP-A-201184) wird die Temperatur des Temperiermediums zyklisch variiert. Hierzu wurden sogenannte Thermocycler beschrieben (US-A-5,038,852 und EP-A-0 488 769). Bei diesem Verfahren wird ein Reaktionsblock aus Metall, in dem sich Ausnehmungen für die Reaktionsgefäße befinden, erhitzt und abgekühlt, um die Temperaturwechselbehandlungen zu erreichen.

In WO 92/07089 ist ein System beschrieben, bei dem die Reaktionsflüssigkeit in einem geschlossenen Kreislauf zwischen Zonen mit Kühl- bzw. Heizelementen hin und her transportiert wird. Das hierfür erforderliche System ist jedoch kompliziert und für einen Gebrauch in der Routine wenig geeignet.

In der älteren, nicht vorpublizierten DE-A-4409436 ist ein Verfahren beschrieben, bei dem ein kombiniertes Heiz-/Kühlelement in das Reaktionsmedium eingetaucht wird und die Temperatur des Reaktionsmediums nur in der unmittelbaren Nähe des Heizelementes geändert wird.

Bei der Durchführung einer Temperaturwechselbehandlung von Probenflüssigkeiten, insbesondere während der Polymerase-Kettenreaktion werden Temperaturen angewandt, bei denen der Dampfdruck des Wassers relativ hoch liegt. Daher schlägt sich üblicherweise Flüssigkeit am Deckel des Reaktionsgefäßes nieder. Da dies jedoch zu einer Aufkonzentrierung der Reaktionskomponenten in der Reaktionsmischung führt, welche nicht kontrollierbar ist, wurde vorgeschlagen, in den Deckel eine Heizung zu integrieren, wodurch eventuell am Deckel niedergeschlagene Flüssigkeitströpfchen wieder in die Gasphase überführt werden können. Diese Deckelheizungen sind jedoch so positioniert, daß sie nur Bereiche erhitzen, die nicht in die Reaktionsmischung eintauchen.

Gegenstand der vorliegenden Erfindung war es, ein alternatives System zur Temperaturwechselbehandlung von Flüssigkeiten bereitzustellen.

Gegenstand der Erfindung ist ein System zur Temperaturwechselbehandlung von nukleinsäurehaltigen Flüssigkeiten in einem Gefäß, wobei das System ein wiederverwertbares Temperierelement, ein Gefäß und ein disposibles Heizelement enthält, wobei das Heizelement integrierter Bestandteil des Gefäßes oder eines Deckels des Gefäßes ist und zur Durchführung der Behandlung in die Flüssigkeit eintaucht.

Ebenfalls Gegenstand der Erfindung ist ein Verfahren zur Behandlung von Nukleinsäuren in einer Flüssigkeit unter Einstellung von 2 oder mehr Temperaturen mittels eines Temperier- und eines Heizelements, wobei das Temperierelement Teil eines wiederverwertbaren Gerätes und das Heizelement Teil einer disposiblen Vorrichtung ist.

Das erfindungsgemäße System ist zum Einsatz in solchen Verfahren gedacht, bei denen eine Flüssigkeit oder Segmente davon auf verschiedene Temperatumiveaus gebracht werden muß. Dies ist beispielsweise erforderlich, wenn Vorgänge, die in der Flüssigkeit ablaufen sollen, z. B. chemische oder vorzugsweise enzymatische Reaktionen, nur oder bevorzugt bei bestimmten Temperaturen ablaufen. Weitere Vorgänge, die temperatursensitiv sind, sind wie oben geschildert die Trennung von zueinander komplementären Nukleinsäuresträngen durch Erwärmung bzw. Inkubation der Flüssigkeit auf eine Temperatur oberhalb des entsprechenden Schmelzpunktes (Tₘ) und die Bildung von Hybriden aus Nukleinsäuren, die im wesentlichen zueinander komplementär sind bei Temperaturen, die unterhalb des Schmelzpunktes, bevorzugt mehr als 15 °C unterhalb des Schmelzpunktes liegen, die sogenannte Hybridisierung. Ein weiterer Vorgang, der die Behandlung bei einer erhöhten Temperatur erfordert, ist der Aufschluß von Zellkompartimenten. Des weiteren können erhöhte Temperaturen zur gezielten Zerstörung von in der Flüssigkeit enthaltenen, temperaturinaktivierbaren Inhaltsstoffen benutzt werden, z. B. auch zur Inaktivierung von für den Aufschluß benutzten Enzymen, z. B. Proteinasen. Das erfindungsgemäße System erlaubt die Einstellung der jeweils erforderlichen oder gewünschten Temperatur, unabhängig davon, wie oft ein Temperaturwechsel erforderlich ist. Es ist daher auch möglich, einige oder mehrere dieser Schritte hintereinander und abwechselnd mehrfach, z. B. zyklisch, durchzuführen.

Unter einer Temperaturwechselbehandlung einer Flüssigkeit soll im Sinne der Erfindung eine Behandlung der Flüssigkeit verstanden werden, bei denen die Flüssigkeit so behandelt wird, daß Vorgänge, die in der Flüssigkeit ablaufen sollen, bei unterschiedlichen Temperaturen ablaufen können. Hierbei sind sowohl zeitliche Temperaturprofile als auch örtliche Temperaturprofile umfaßt.

Ein prominentes Beispiel für die mehrfache Durchführung von Behandlungen bei unterschiedlichen Temperaturen ist die Amplifikation von Nukleinsäuren gemäß der Polymerase-Kettenreaktion. Diese Reaktion ist in der Fachwelt mittlerweile vielfach und in verschiedensten Modifikationen beschrieben. Ein prominentes Beispiel einer solchen Offenbarung ist US-A-4,683,202. Essentielles Merkmal der Polymerase-Kettenreaktion (PCR) ist die mehrfache Durchführung von Temperaturzyklen, welche eine Behandlung bei hohen Temperaturen, z. B. in einem Bereich zwischen 90 und 95 °C zur Einzelsträngigmachung eventuell vorhandener doppelsträngiger Nukleinsäuren, eine Behandlung bei niedrigen Temperaturen, z. B. im Bereich zwischen 50 und 65°C, zur Hybridisierung von Primern an die zu amplifizierenden Nukleinsäuresequenzen und einer mittleren Temperatur, z. B. in einem Bereich zwischen 70 und 75 °C zur optimalen Verlängerung des Primers unter Verwendung der zu amplifizierenden Nukleinsäure als Matrize. Die Möglichkeiten zur Variation der Temperaturzyklen sind beispielsweise auch beschrieben in EP-A-0 511 712.

Nukleinsäuren, die einer erfindungsgemäßen Behandlung unterzogen werden können, sind alle natürlich vorkommenden oder davon abgeleiteten, Nukleobasen enthaltende Biopolymere oder Analoge hierzu, die durch Modifizierung entweder der Base oder des Zuckerphosphatbackbones erhältlich sind. Die Nukleinsäuren können in der Flüssigkeit in gelöster Form, in zellgebundener Form und in an eine feste Oberfläche gebundener Form (immobilisiert), z. B. an Partikeln, vorliegen. Bevorzugt liegen die Nukleinsäuren zumindest während eines Schrittes der Temperaturwechselbehandlung in gelöster Form vor. Es ist möglich, die Nukleinsäuren von einer immobilisierten Form in die gelöste Form zu überführen und umgekehrt, z. B. durch Erhitzung von über eine immobilisierte Sonde an eine Oberfläche gebundener Nukleinsäuren.

Als Flüssigkeiten eignen sich prinzipiell besonders alle nukleinsäurehaltigen Flüssigkeiten, z. B. Proben, die direkt aus ihrer ursprünglichen Umgebung entnommen wurden. Als Flüssigkeiten sind jedoch insbesondere solche geeignet, die eine gewisse Aufbereitung erfahren haben, z. B. einen Schritt zur Entfernung bestimmter Probenbestandteile (z. B. eine spätere Analyse störender Bestandteile), zur Verflüssigung der Probe (z. B. bei hochviskosen Proben), einer Konzentration oder Verdünnung der Probe, eines Aufschlusses, jedoch auch zur Isolierung der Nukleinsäuren aus einer ursprünglichen Probe (z. B. zur Vorreinigung).

Als Proben kommen insbesondere Körperflüssigkeiten, wie Blut, Urin, Sputum oder Abstriche in Betracht.

Das Gefäß, in dem die Temperaturwechselbehandlung vorgenommen wird, ist vorzugsweise hergestellt aus einem Material, welches die Temperaturwechselbehandlung ohne Veränderung der Form oder Abgabe von Materialbestandteilen an die Flüssigkeit übersteht. Besonders geeignet hierfür sind Kunststoffe, z. B. Polypropylen oder Polystyrol. Die Größe des Gefäßes wird so gewählt, daß die Probe und eventuell zugegebene Reagenzien und das Heizelement hineinpassen. Besonders geeignet sind z. B. von Eppendorff-Hütchen abgeleitete Gefäße, die jedoch bevorzugt keinen im Material mit dem Hütchen verbundenen Deckel aufweisen. Solche Gefäße sind kommerziell erhältlich oder auf einfache Weise durch Spritzgußverfahren herstellbar.

Weiterer wesentlicher Bestandteil des Systems ist ein Deckel, mit welchem das Gefäß verschlossen werden kann. Er sollte dazu geeignet sein, den Ein- und Austrag von Kontaminationen, z. B. über Aerosole, aus dem Gefäß und in das Gefäß hinein in Grenzen zu halten. Auch er besteht aus einem im wesentlichen temperaturbeständigen Material, wie für das Gefäß angegeben.

Ein Temperierelement ist ein Gegenstand, der aktiv auf eine gewünschte Temperatur gebracht werden kann, bevorzugt ein Kühlelement. Das Kühlelement im Sinne der Erfindung ist ein Gegenstand, welcher aktiv gekühlt wird und Wärme direkt oder indirekt aus der Flüssigkeit aufnehmen kann. Er umfaßt nicht das Gefäß. In einer ersten Ausführungsform ist das Kühlelement beispielsweise ein Metallblock, welcher über Peltierelemente (Trockenkühlung) oder über gekühlte Flüssigkeiten (Flüssigkeitskühlung) gekühlt werden kann. Sofern es sich um einen Metallblock handelt, ist dieser bevorzugt an die äußere Kontur des Gefäßes angepaßt. Die Anpassung kann z. B. dadurch geschehen, daß das Kühlelement hohlzylindrische Ausnehmungen aufweist, in welche die Gefäße eingedrückt werden können. Je besser die Anpassung des Kühlelementes an die Außenform des Gefäßes ist, desto besser ist auch die Kühlwirkung. In einer anderen Ausführungsform ist das Kühlelement ein bevorzugt metallisches Bauelement, welches durch eine Öffnung, bevorzugt eine durch den Deckel verschließbare Öffnung, in das Gefäß, besonders bevorzugt bis unter den Flüssigkeitspegel, hineinragt. Hier ist insbesondere eine Kühlung über Peltierelemente bevorzugt. Das Kühlelement ist in diesem Fall bevorzugt über eine Folie, z. B. aus Teflon oder Polyester, vor direkter Kontamination durch die Flüssigkeit geschützt. Die Folie wird nicht als Teil des Kühlelementes angesehen, da sie nicht wiederverwertbar ist. Das Kühlelement kann jedoch auch ein Wasserbad sein, in welches das Gefäß hineinragt. Hierbei ist der Wärmeübergang vom flüssigen Kühlmedium über das Gefäß in die Reaktionsmischung besonders direkt. Ein Temperierelement im Sinne der Erfindung kann jedoch durchaus auch Heizfunktion haben, wenn die Temperaturwechselbehandlung in der Flüssigkeit eine untere Grenztemperatur erfordert, die wesentlich, d. h. mehr als 5 K, oberhalb der Raumtemperatur liegt. In diesem Fall kann es sein, daß die Wärmeabfuhr durch das Temperierelement an die Umgebung so groß ist, daß zum Erhalt der unteren Schwellentemperatur sogar Wärme zugeführt werden muß. Dennoch liegt die minimale während des Verfahrens erreichte Temperatur des Temperierelements immer unterhalb der maximalen während des Verfahrens erreichten Temperatur des Heizelementes.

Unter Wiederverwertbarkeit des Kühlelementes wird die Möglichkeit verstanden, das selbe Kühlelement zur Behandlung mindestens einer weiteren Flüssigkeit zu benutzen. Diese weitere Flüssigkeit hat bevorzugt eine von der ersten Flüssigkeit unterschiedliche Zusammensetzung, so daß darauf geachtet werden muß, daß eine Kontamination der weiteren Flüssigkeit durch die erste Flüssigkeit minimiert ist. Aus diesem Grund ist die Ausführungsform, in der das Kühlelement das Gefäß von außen kühlt, bevorzugt.

Ein Heizelement im Sinne der Erfindung ist ein Gegenstand, der aktiv geheizt wird und dessen Wärmeentwicklung zur Erwärmung der zu behandelnden Flüssigkeit benutzt wird. Es kann sich hierbei um ein mehrkomponentiges Heizelement handeln. Bevorzugt enthält das Heizelement einen Metalldraht oder eine Metallfolie, z. B. aus Gold, oder ein Graphitelement. Solche Heizelemente sind dem Fachmann bekannt. Die Heizleistung des Heizelements wird so ausgelegt, daß die gewünschte Temperatur der Flüssigkeit in gewünschter Zeit erreicht wird. Dies kann beispielsweise durch Variation der Größe des Heizelementes, der verwendeten Materialien und der Stromzufuhr erreicht werden.

Disposibel im Sinne der Erfindung ist ein Element, welches nach Durchführung eines Verfahrens zur Temperaturwechselbehandlung einer bestimmten Flüssigkeit weggeworfen wird. Es wird nicht für die Temperaturwechselbehandlung von weiteren Flüssigkeiten benutzt, die einer unabhängigen Temperaturwechselbehandlung unterzogen werden sollen. Insbesondere wird in der Analytik solcher Flüssigkeiten das Heizelement zwischen jeder Analyse verworfen. Aus diesem Grund sind einfach gebaute und kostengünstig hergestellte Heizelemente bevorzugt.

Ein integrierter Bestandteil eines Bauteiles im Sinne der Erfindung ist ein Bestandteil, welcher nicht ohne Zerstörung entweder des Heizelementes oder des Bauteiles (des Gefäßes oder des Deckels) von diesem Bauteil getrennt werden kann. Besonders bevorzugt ist das Heizelement in das Gefäß oder den Deckel eingegossen, was besonders Vorteile für die spritzgußtechnische Herstellung mit sich bringt. In einer ersten Ausführungsform kann das Heizelement in das Gefäß integriert sein. Hierbei sollte darauf geachtet werden, daß das Heizelement im Bereich der Flüssigkeitsaufnahme lokalisiert ist, d. h. zum Beispiel am Boden des Gefäßes oder an der Seitenwand des Gefäßes, die mit der (zu heizenden) Flüssigkeit in Kontakt kommt. In der bevorzugten Ausführungsform, in welcher das Heizelement ein integrierter Bestandteil des Deckels ist, ist das Heizelement bevorzugt an der Innenseite des Deckels befestigt und ragt bei aufgesetztem Deckel in das Gefäß hinein, insbesondere bis unter den Flüssigkeitspegel. Das Heizelement oder Anschlüsse, z. B. für Strom, ragen durch den Deckel auf die Außenseite des Deckels hinaus und können mit Kopplungselementen an ein wiederverwertbares Gerät zur Versorgung des Heizelementes mit Strom und gegebenenfalls zur Regulierung der Heizleistung versehen sein.

Das Eintauchen des Heizelementes in das Gefäß geschieht so, daß die aufzuheizenden Teile der Flüssigkeit eine ausreichende Wärme erhalten können. Das Heizelement ragt daher bevorzugt über seine gesamte Höhe in die Flüssigkeit hinein.

Zu dem erfindungsgemäßen System können neben den essentiellen Bestandteilen Gefäß, Deckel, Heizelement und Kühlelement noch weitere, für die Durchführung von Temperaturwechselbehandlungen von Flüssigkeiten und gegebenenfalls anschließenden Weiterbearbeitungsschritten, geeignete Elemente enthalten sein. Hierzu gehören insbesondere Bauelemente zur Versorgung der Heiz- und Kühlelemente mit Kühlmittel bzw. Strom, Elemente zur Regelung der Temperatur, Elemente zur Messung der Temperatur, Einheiten zum Transport von Gefäßen, Elemente zur Pipettierung von Flüssigkeiten in das Gefäß und aus dem Gefäß heraus und Elemente zur Steuerung des Gesamtsystems. Bevorzugt enthält das System eine Vielzahl von Gefäßen und Deckeln, so daß es zur Behandlung einer Vielzahl von (insbesondere nukleinsäurehaltigen) Flüssigkeiten in Serie oder/und parallel geeignet ist.

Bezüglich der Heizung und Kühlung der Flüssigkeit sind zwei besondere Ausfiihrungsformen möglich. In einer ersten Ausführungsform ist das Heizelement in Intervallen aktiv, z. B. wird der Flüssigkeit Heizleistung kurzzeitig (z. B. nur wenige Bruchteile von Sekunden) zugeführt, während die Kühlung permanent erfolgt. Hierdurch bilden sich in der Flüssigkeit bevorzugt zeitlich aufeinanderabfolgende unterschiedliche Temperaturgradienten, wobei die Temperaturen in der Nähe des Kühlelementes im wesentlichen konstant bleiben, während die Temperatur der Flüssigkeit in der Nähe des Heizelementes stärker variiert. Hierdurch kann beispielsweise erreicht werden, daß in unterschiedlichen Regionen im Gefäß unterschiedliche Reaktionen ablaufen. Beispielsweise würde im Fall der Erhitzung des Heizelements auf für die Denaturierung von Nukleinsäuren erforderliche Temperaturen (oberhalb des Tₘ-Wertes) eine Denaturierung nur in der Nähe des Heizelementes stattfinden, wonach die denaturierten Nukleinsäuren in Bereiche transportiert werden können, in denen eine Hybridisierung mit anderen Nukleinsäuren stattfinden kann. Dieser Transport kann beispielsweise durch Konvektion geschehen, jedoch ist Diffusion bevorzugt.

In einer zweiten, besonders bevorzugten Ausführungsform sind sowohl die Heizung als auch die Kühlung permanent eingeschaltet und bevorzugt konstant. Hierdurch bildet sich ein bei ausreichend langer Einwirkung stabiler Temperaturgradient aus, welcher durch die Wärmeleitfähigkeit der Flüssigkeit sowie durch Diffusion und gegebenenfalls Konvektion in der Flüssigkeit kontrolliert ist. Auch hier können in unterschiedlichen Bereichen des Gefäßes unterschiedliche Reaktionen ablaufen. In dieser Ausführungsform sind bevorzugt alle Komponenten, die an den jeweiligen Reaktionen teilnehmen sollen, in der Flüssigkeit gelöst.

In einer bevorzugten Ausführungsform besteht das System neben einer Vielzahl von Deckeln und Gefäßen im wesentlichen aus einem Temperierblock sowie Elementen zur Bereitstellung und Regelung von elektrischer Energie für den Betrieb des Heizelementes.

Der Temperierblock ist ein vorzugsweise metallischer Körper mit Aufnahmebohrungen für Kunststoffgefäße. Die nötige Temperierleistung kann hierbei entweder durch Verwendung temperierter Flüssigkeiten (Wärmeträger-Flüssigkeit, Umlauf-Kühlung), Einsatz von Peltier-Elementen oder sonstigen bekannten Temperierverfahren zugeführt werden.

Die Abmessungen der Aufnahmebohrungen für die Kunststoffgefäße orientieren sich streng an den Außenabmessungen der Kunststoffgefäße, direkter Kontakt von Temperierblock und Kunststoffgefäß ist für den guten Wärmeübergang erforderlich. Diese konstruktiven Merkmale sind dem Fachmann jedoch geläufig.

Vorzugsweise sollte die Tiefe der Bohrung im Verhältnis 5:1 zum Durchmesser stehen, da dadurch bei guter Ausbildung von Temperaturgradienten eine für das System günstige Umwälzung der Flüssigkeit stattfindet.

Das Kunststoffgefäß, in welchem die kontinuierliche Temperaturwechselbehandlung abläuft, ist vorzugsweise aus Polypropylen mit einer Wandstärke kleiner als 1,0 mm (jedoch abhängig vom Gesamtvolumen der Reaktionslösung).

Bei Reaktionsführungen im Bereich der Anwendungen in der Klinischen Chemie und in der Nukleinsäurediagnostik werden üblicherweise Volumina kleiner als 1 ml eingesetzt. Daraus ergeben sich ca.-Abmessungen für das Gefäß von 8 mm Innendurchmesser und 40 mm Höhe.

Zur Verhinderung von Verunreinigungen der Reaktionslösung und zur Unterbindung von Verdunstungsverlusten wird das Gefäß mit einem Deckel, ebenfalls im Spritzguß aus Polypropylen gefertigt, verschlossen.

Das disposible Heizelement besteht bevorzugt im wesentlichen aus einem Kunststoff-Formkörper, den Elektroanschlüssen sowie der Heizleiterfolie. Die Abmessungen des disposiblen Heizelementes sind angepaßt an die Abmessungen des Reaktionsgefäßes.

Eine bevorzugte Ausführungsform des disposiblen Heizelementes besteht darin, daß in ein aus Deckel und Halterungen bestehendes, im Spritzgußverfahren gefertigtes Kunststoffteil, eine vorgefertigte Anordnung von Heizleiterfolie und Kontaktierungen integriert ist.

Die Heizleiterfolie ist bevorzugt eine 20 µm dicke Goldfolie. Der für das Spritzgußteil verwendete Kunststoff ist Polypropylen. Die Fläche des aktiven Heizelementes beträgt vorzugsweise ca. 60 mm², das untere Ende des Elementes ragt bis zum Gefäßboden in das Reaktionsgefäß.

Das oben geschilderte System zur Temperaturwechselbehandlung von nukleinsäurehaltigen Flüssigkeiten kann vorteilhaft in vieler Weise eingesetzt werden.

Ebenfalls Gegenstand der Erfindung ist daher ein Verfahren zur Behandlung von Nukleinsäuren in einer Flüssigkeit unter Einstellung von zwei oder mehr Temperaturen mittels eines Kühl- und eines Heizelementes, wobei das Kühlelement Teil eines wiederverwertbaren Gerätes und das Heizelement Teil einer disposiblen Vorrichtung ist. Die oben genannten bevorzugten Merkmale gelten auch für dieses Verfahren. Als besonders zweckmäßig hat sich die Verwendung des erfindungsgemäßen Verfahrens in thermozyklisch geführten Reaktionen erwiesen. Bei solchen Verfahren finden bei unterschiedlichen Temperaturen unterschiedliche Reaktionen statt. Durch Unterwerfung der Reagenzien unter bestimmte Temperaturen können die Reaktionen ablaufen. Dies kann einerseits, wie oben beschrieben, durch zeitliche Variation des Temperaturprofils in der Reaktionsmischung durch Erhöhung oder Erniedrigung der Heiz- bzw. Kühlleistung geschehen, andererseits jedoch auch durch Anlegen eines konstanten Temperaturprofils zwischen den beheizten Bereichen und den gekühlten Bereichen. Übergangsformen, z. B. bewirkt durch Konvektionen der Mischung sind denkbar.

Wesentlich ist, daß nach dem erfindungsgemäßen Verfahren die Reaktanten der gewünschten Reaktion nacheinander unterschiedlichen Temperaturen ausgesetzt werden, so daß die gewünschten unterschiedlichen Reaktionen ablaufen können. Während der Gesamtbehandlungszeit kann beispielsweise für zyklische Reaktionen durch Steuerung der Heiz- bzw. Kühlleistung erreicht werden, daß die Reaktanten zyklisch unterschiedlichen Reaktionsbedingungen unterworfen werden und daher Reaktionszyklen nacheinander durchgeführt werden. Bei Anlegen eines relativ konstanten Temperaturgradienten an der Reaktionsmischung erfolgt die Zyklusbehandlung bevorzugt durch Diffusion der Reaktionspartner aus einem ersten Raumsegment der Reaktionsmischung mit einer ersten Temperatur in ein zweites Raumsegment mit einer zweiten Temperatur. Durch Diffusion in ein Raumsegment mit einer Temperatur, wie sie die darauffolgende Reaktion (z. B. erneut bei der ersten oder einer dritten Temperatur) benötigt findet die Zyklusführung statt. Da Diffusionsvorgänge üblicherweise relativ langsam stattfinden, ist es bevorzugt, einen relativ steilen Temperaturgradienten zu wählen, d. h. dafür zu sorgen, daß das Temperaturgefälle zwischen Heizelement und Kühlelement auf eine relativ kurze Strecke beschränkt wird. Typische Wegstrecken zwischen Heiz- und Kühlelement sind wenige Millimeter.

Ein typisches Beispiel für ein Verfahren zur Temperaturwechselbehandlung von Nukleinsäuren ist die Amplifikation von Nukleinsäuren oder Teilen davon. Ein Beispiel hierfür ist die Polymerasekettenreaktion, wie sie in US-A-4,683,202 beschrieben ist. Ein weiteres Beispiel ist die Ligasekettenreaktion.

Ein weiterer Gegenstand der Erfindung ist ein Verfahren zum Nachweis einer Nukleinsäure einer Probe durch
a) Freisetzen der Nukleinsäure, auf der der Nachweis beruhen soll, aus Kompartimenten, in denen sie enthalten ist, in einem Gefäß in einem erfindungsgemäßen System,
b) Vermehrung von Sequenzinformationen, die auf der Anwesenheit der Nukleinsäure in dem Gefäß beruht und
c) Nachweis der Sequenzinformationen.

Das erfindungsgemäße System kann somit zur deutlichen Vereinfachung von Nukleinsäurenachweisverfahren eingesetzt werden. Besonders bevorzugt wird die Flüssigkeit mit Ausnahme von Mischvorgängen nicht innerhalb des Gefäßes von einem Ort an einen anderen transportiert.

Die Freisetzung der Nukleinsäuren kann auf im Prinzip bekannte Weise geschehen. Übliche Behandlungen enthalten die Lyse von Zellwänden, z. B. durch geeignete Reagenzien, wie Proteinase K, Detergenzien oder Alkali, oder/und durch Hitze. Dies bewirkt, daß die Nukleinsäuren in Lösung und zugänglich für Reagenzien zur Weiterverarbeitung vorliegen. Dieser Schritt findet in einem Gefäß statt, das inert ist gegenüber den Reaktionsbedingungen der Freisetzung und dem folgenden Schritt b), z. B. Polypropylen. In dem selben Gefäß wird nun Sequenzinformation, die auf der Anwesenheit der Nukleinsäure in dem Gefäß beruht, vermehrt, z. B. durch Amplifikation eines Teilbereiches der freigesetzten Nukleinsäuren. Dies kann beispielsweise geschehen durch die Polymerasekettenreaktion.

Unter Sequenzinformation wird die Abfolge von Basen verstanden, z. B. die (Nukleotidsequenz) eines Teils oder der Gesamtheit der nachzuweisenden Nukleinsäure.

Prinzipiell kann die Sequenzinformation jedoch auch in einer Nukleotidsequenz bestehen, die durch eine Bindereaktion an die nachzuweisende Nukleinsäure gekoppelt und anschließend vermehrt wurde. Es kann sich hierbei z. B. um eine sogenannte Signalamplifikation handeln. Wesentlich für den Gegenstand der Erfindung ist, daß die Reaktionen der Schritte a) und b) in dem selben Gefäß stattfinden. Schritt b) kann beispielsweise dadurch gestartet werden, daß die nukleinsäurehaltige Flüssigkeit in dem Gefäß einer Temperaturwechselbehandlung mit Hilfe des oben genannten wiederverwertbaren Temperierelements, insbesondere Kühlelements, und dem disposiblen Heizelement unterworfen wird. Dies kann bevorzugt dadurch geschehen, daß das Gefäß während der Schritte a) und b) in dem wiederverwertbaren Kühlelement aufbewahrt wird und zur Durchführung des Schrittes b) das disposible Heizelement in das Gefäß eingeführt wird. Wenn das disposible Heizelement in einen Deckel integriert ist, kann dieser auch schon während des Schrittes a) auf dem Gefäß befindlich sein und zur Hitzebehandlung während und nach Freisetzung der Nukleinsäure zur Hitzebehandlung eingesetzt werden.

Der Nachweis der Sequenzinformation kann nach prinzipiell bekannten Verfahren geschehen, z. B. durch Überführung der Reaktionsmischung aus Schritt b) in ein Gefäß, in dem die entstandenen Nukleinsäuren, bevorzugt über eine Hybridisierungsreaktion, nachgewiesen werden. Eine mögliche Versuchsführung benützt das sogenannte Sandwich-Prinzip, wie es in EP-B-0 079 139 beschrieben ist. Dieses Verfahren benutzt eine zu der vermehrten Sequenzinformation komplementäre Fangsonde, die entweder an eine feste Phase gebunden ist oder gebunden werden kann und eine Nachweissonde, die markiert ist und zu einem anderen Teil der vermehrten Sequenzinformation komplementär ist. Die Bildung des Komplexes aus Sonden und vermehrter Sequenzinformation enthaltene Nukleinsäure wird zum Zeichen der Anwesenheit von Nukleinsäuren in der Probe benutzt. Durch die Vermeidung einer Überführung der Nukleinsäure von einem Gefäß in ein anderes wird die Gefahr einer Kontamination der Reaktionsmischung und der Umgebung stark reduziert. Darüber hinaus ist das Verfahren natürlich sehr viel einfacher und unter Verwendung weniger Geräte durchführbar.

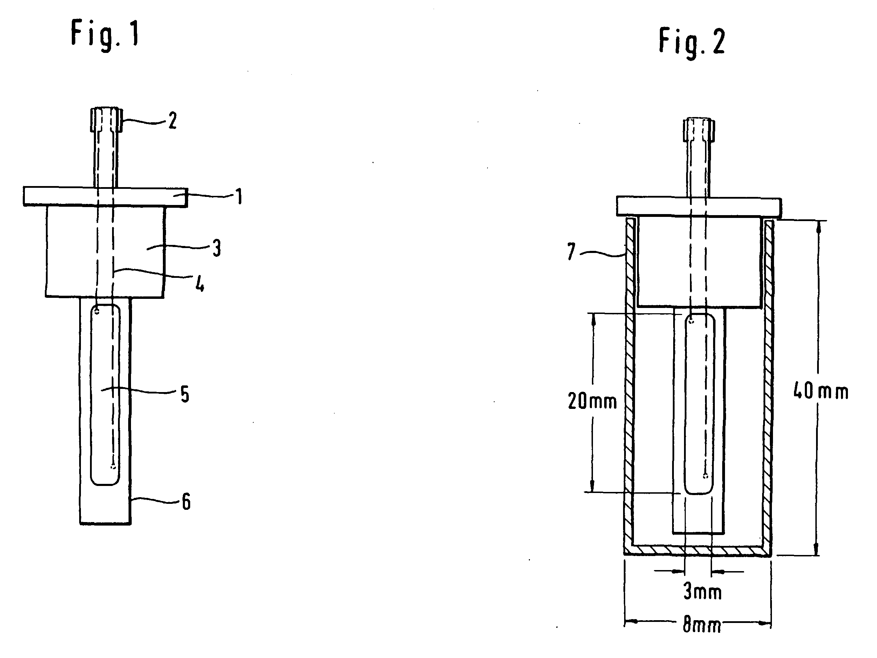

In Figur 1 ist ein erfindungsgemäßer Deckel (1) mit integriertem Heizelement gezeigt. Es ist erkennbar, daß der Deckel ein Verschlußteil (3) aufweist, welcher der Form der Öffnung des zu verschließenden Gefäßes angepaßt ist. Der Verschlußteil setzt sich fort in einer Kunststoffhalterung (6) für das Heizelement (5). An der Oberfläche dieser Kunststoffhalterung ist das Heizelement so befestigt, daß die Zuleitungen (4) für das Heizelement im Inneren der Kunststoffhalterung bzw. des Verschlußteiles zu liegen kommen und an einem Ende in Elektrokontakte (2) zum Anschluß an eine Stromversorgung enden.

In Figur 2 ist der Deckel in auf ein Gefäß aufgesetzter Form gezeigt. In Figur 2 sind auch Außenmaße für ein Gefäß und den in Figur 1 gezeigten Deckel angegeben. Diese Außenmaße sind für die Durchführung von erfindungsgemäßen Verfahren, z. B. Durchführung von Amplifikationsverfahren, geeignet, können jedoch von einem Fachmann insbesondere an abweichende Flüssigkeitsmengen auf einfache Weise angepaßt werden. Das Gefäß ist mit dem Bezugszeichen (7) gekennzeichnet.

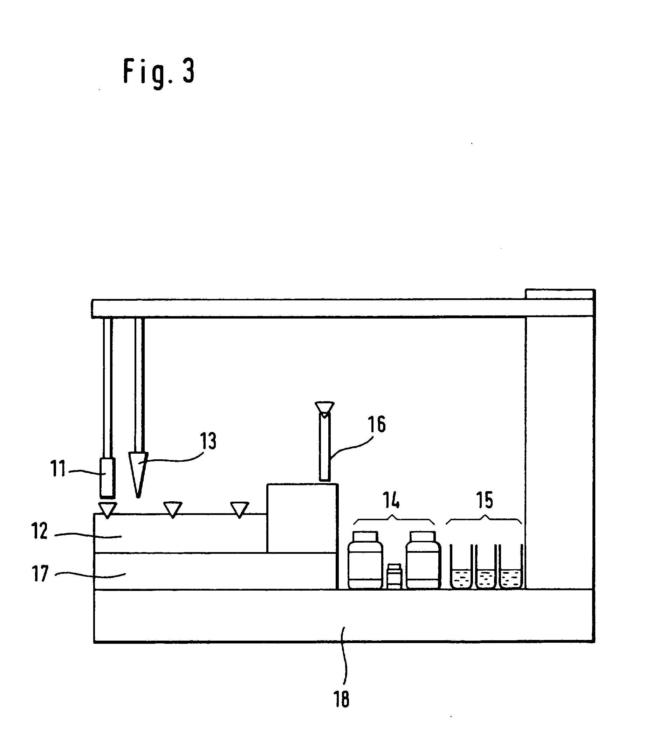

In Figur 3 ist ein erfindungsgemäßes System mit Probenvorbereitungsmodul 17 gezeigt, mit welchem eine Vorbereitung von nukleinsäurehaltigen Flüssigkeiten für die Amplifikation und die Amplifikation selbst durchgeführt werden kann. In dieser Figur kann der Tophandler (Deckelhandler 11) den Deckel der Figur 1 (Top 1) ergreifen und auf bereitstehende Reaktionsgefäße (Disposable-Devices 12) aufsetzen. In den Tophandler sind darüber hinaus Kontakte für die Stromversorgung der Deckelheizung integriert. Mit Hilfe der Pipettiereinheit und Pipettenspitzen (Disposable-Tips 13) können Reagenzien 14 oder/und Probenflüssigkeiten 15 in die Reaktionsgefäße 7 (hier Disposable-Devices 12) überführt werden. Sobald der erfindungsgemäße Deckel ausgebraucht ist, kann er in ein Abfallgefäß 16 überführt werden (solid phase disposable with top). Die gesamten Vorgänge werden bevorzugt in einem Gerät durchgeführt, in dem in alle 3 Raumrichtungen (x, y, z) Pipettier- und Transportvorgänge stattfinden können (z. B. Laborroboter 18).

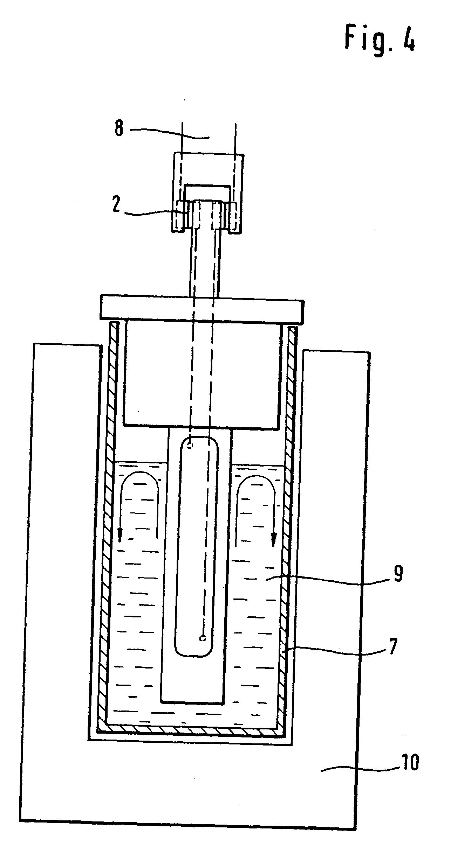

In Figur 4 ist ein System mit Stromanschluß (8), Elektrokontakten (2), Reaktionsgemisch (9), welches durch die Wärmeentwicklung des Heizelements konvektiv gemischt wird, Gefäß (7) und Kühlblock (10) gezeigt.

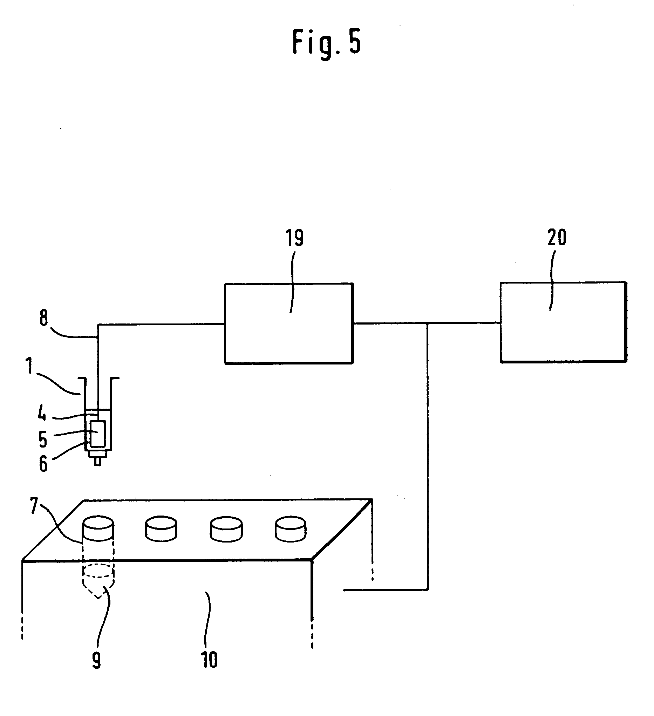

In Figur 5 ist ein schematischer Aufbau einer Versuchsdurchführung mit Temperaturwechselbehandlung gezeigt.

### Bezugszeichenliste

- 1.: Deckel mit disposiblem Heizelement
- 2.: Elektrokontakte
- 3.: Verschlußteil des Deckels
- 4.: Elektrozuleitung für das Heizelement (ins Innere des Kunststoffs eingegossen)
- 5.: Heizelement (Goldfolie)
- 6.: Kunststofihalterung für das Heizelement
- 7.: Gefäß
- 8.: Stromanschluß
- 9.: Reaktionsmischung
- 10.: Kühlelement
- 11.: Deckelhandler (Ergreifen, Abnehmen, Aufsetzen des Deckels, Stromzufuhr über Kontakte)
- 12.: Disposable-Devices (enthalten eine Vielzahl, z. B. 16, Gefäße, die miteinander verbunden sind)
- 13.: Pipettenspitzen an einem Pipettierarm des Gerätes
- 14.: Reagenzien in Gefäß
- 15.: Probenflüssigkeit in Gefäß
- 16.: Abfallgefäß für Deckel
- 17.: Probenvorbereitungsmodul (Aufnahme für Gefäße, Temperierblock)
- 18.: Laborroboter (mit Regelung der Transport- und Temperierschritte, Ablaufsteuerung)
- 19.: Steuereinheit für Heizelement
- 20.: Regel- und Auswerterechner für Gesamtverfahren

### Beispiel 1

### Erstellung eines Systems für die Durchführung einer DNA-Analyse

Das System ist aufgebaut aus einem Wasserbad, das auf eine Temperatur von 57°C geregelt ist. Über der Wasseroberfläche ist eine Lochplatte in einem Abstand befestigt, daß das Reaktionsgefäß gemäß Figur 2 zu ungefähr der Hälfte in das Wasser eintaucht. Ein Rand am Gefäß verhindert, das Abrutschen des Gefäßes in das Wasserbad. Die Aufnahmebohrungen der Lochplatte sind daher nur geringfügig größer als 8 mm. Das Kunststoffgefäß ist aus Propylen mit einer Wandstärke von 0,4 mm (Figur 2).

Das eingesetzte Heizelement ist schematisch in Figur 1 dargestellt. Es handelt sich um ein im Spritzgußverfahren gefertigtes Kunststoflieil, in welches eine 20 µm dicke Goldfolie und Heizleitungen so integriert sind, daß die Goldfolie auf einer Folienseite von der Flüssigkeit benetzt werden kann.

### Beispiel 2

### Durchführung einer DNA-Analyse mit statischen Temperaturgradienten

### 1. Probenvorbereitung/DNA-Isolierung

Humane Leukozyten-DNA wurde nach folgender Methode aus Vollblut unter Verwendung des QIAamp Blood Kit (Best. Nr. 29104) der Firma Quiagen (BRD, Postfach, 40719 Hilden) isoliert.

In einem 2 ml Eppendorf-Gefäß werden 200 µl EDTA-antikoaguliertes Vollblut, 25 µl Proteinase-K-Lösung (19 mg/ml) und 200 µl Probenaufschluß/Bindungspuffer pipettiert. Die Probe wird sofort mittels Vortex® geschüttelt, um das sich bildende Pellet zu resuspendieren. Die Probe wird für 10 Minuten bei 70 °C erhitzt, danach auf Raumtemperatur abgekühlt und mit 210 µl Isopropanol aufgestockt. Die Probe wird in ein QIAamp "spin-column" überführt. Das "spin-column" ist ein nach unten offenes Zentrifugationsdevice/Röhrchen, an dessen Boden sich ein Glasfaservlies befindet.

Das "spin-column" wird auf ein 2 ml Probenauffanggefäß (2 ml Eppendorf-Gefäß) gesteckt und in einer Tischzentrifuge bei 6.000 x g (= 8.000 rpm) für 1 Minute zentrifugiert. Das Filtrat wird verworfen und in das "spin-column" 500 µl Waschpuffer pipettiert. Es wird erneut 1 Minute bei 6.000 x g zentrifugiert. Das Filtrat wird verworfen und der Waschvorgang nochmals wiederholt.

Danach werden 200 µl Elutionslösung (10 mM Tris/HCl, 1 mM EDTA, pH 8,0) in das "spin-column" pipettiert und die gebundene DNA durch erneute Zentrifugation (1 Minute bei 6.000 x g) vom Glasfaservlies elutiert.

Die gereinigte DNA wird mittels Extinktionsmessung am Photometer bei 260 nm und 280 nm und mittels Gelelektrophorese charakterisiert. Aus 200 µl Vollblut (ca. 5 x 10⁶ Leukozyten pro ml) werden typischerweise 6 µg DNA in 200 µl Elutionslösung (entspricht ca. 30 ng DNA/µl) mit einem Extinktionskoeffizienten A₂₆₀/A₂₈₀ von 1,7 - 1,9 erhalten (eine Extinktion von 1.000 mE bei 260 nm entspricht einem DNA-Gehalt der Probe von 50 ng/µl.)

Die Fragmentgröße der eluierten DNA liegt zwischen 1 und 50 Kbp, hauptsächlich zwischen 20 und 40 Kbp, bestimmt mittels Gelelektrophorese an einem 1%igen Agarosegel (Ethidiumbromid-Färbung).

### 2. Amplifikation/Vervielfältigung der DNA

Mittels zweier spezifischer Primer wird eine Sequenz aus dem human-tPA-Gen (tPA = tissue-type plasminogen activator) amplifiziert. Die Sequenzen der verwendeten Primer sind:
Forward (d. h. "upstream")
   SEQ.ID.NO. 1: 5'-AGA CAG TAC AGC CAG CCT CA-3'
Reverse (d. h. "downstream")
   SEQ.ID.NO.2: 5'-GAC TTC AAA TTT CTG CTC CTC-3'

Bei Verwendung dieses Primer-Paares entsteht ein Amplifikat mit einer Länge von 375 bp.

Es wird folgender Mastermix in ein oben beschriebenes (PCR)-Reaktionsgefäß aus Polypropylen pipettiert:

| | |
|---|---|
| 10 µl | 10fach PCR-Puffer mit MgCl₂ (100 mM Tris Hcl pH 8,9; 500 mM KCl, 15 mM MgCl₂) |
| 2 µl | 10 mM dNTP-Mix (d. h. je 10 mM dATP, dGTP, dCTP und dTTP) |
| 0,5 µl | Taq-Polymerase (5 U/µl) |
| 1 µl | Forward-Primer (30 µM, Sequenz siehe oben) |
| 1 µl | Reverse-Primer (30 µM, Sequenz siehe oben) |
| 82,5 µl | autoklaviertes, bidestiliertes Wasser |

Sämtliche zur Amplifikation verwendeten Reagenzien (mit Ausnahme der Primer) stammen aus dem PCR Core Kit (Best. Nr. 1578 553) der Fa. Boehringer Mannheim.

Der Mastermix (Σ = 97 µl) wird kurz gevortext, in einer Tischzentrifuge kurz zentrifugiert und danach 3 µl DNA-haltige Probe (aus Punkt 1, DNA-Gehalt ca. 30 ng/µl) dazupipettiert. Das PCR-Gefäß wird in einen erfindungsgemäßen Heiz-/Kühlblock gestellt und mit einem ein disposables Heizelement enthaltenden Deckel verschlossen.

Das Heizelement des Deckels ist so angebracht, daß der Heizdraht zu zwei Dritteln in den PCR-Mix ragt. Das Heizelement wird an die Spannungsversorgung angeschlossen und der PCR-Mix für ca. ½ Stunde so inkubiert, daß sich am Heizdraht im Tube eine Temperatur von 95 °C und an der Tube-Innenwand eine Temperatur von 58 °C einstellt.

Nach beendeter Amplifikation wird der PCR-Mix gemäß Punkt 3 analysiert.

### 3. Analyse des DNA-Amplifikates

In die Probenauftragstasche eines 1 %igen Agarosegels werden 10 µl des PCR-Amplifikates aus Punkt 2 aufgegeben. In eine benachbarte Probenauftragstasche werden 800 ng des Boehringer DNA-Längenstandards VI (Best. Nr. 1062 590, Fragmentgröße 2176 bp bis 154 bp) aufgetragen.

Das Gel wird für 2 Stunden im Spannungsfeld entwickelt und danach auf einem UV-Tisch analysiert.

Bei Anwesenheit von humaner Leukozyten-DNA im Mastermix ist im Gel eine intensive DNA-Bande sichtbar (375 bp), deren Lage zwischen der 394 bp-Bande und der 298 p-Bande des Längenstandards VI liegt.

### Beispiel 3

### Temperaturwechselbehandlung mit periodisch wechselnden Temperaturgradienten

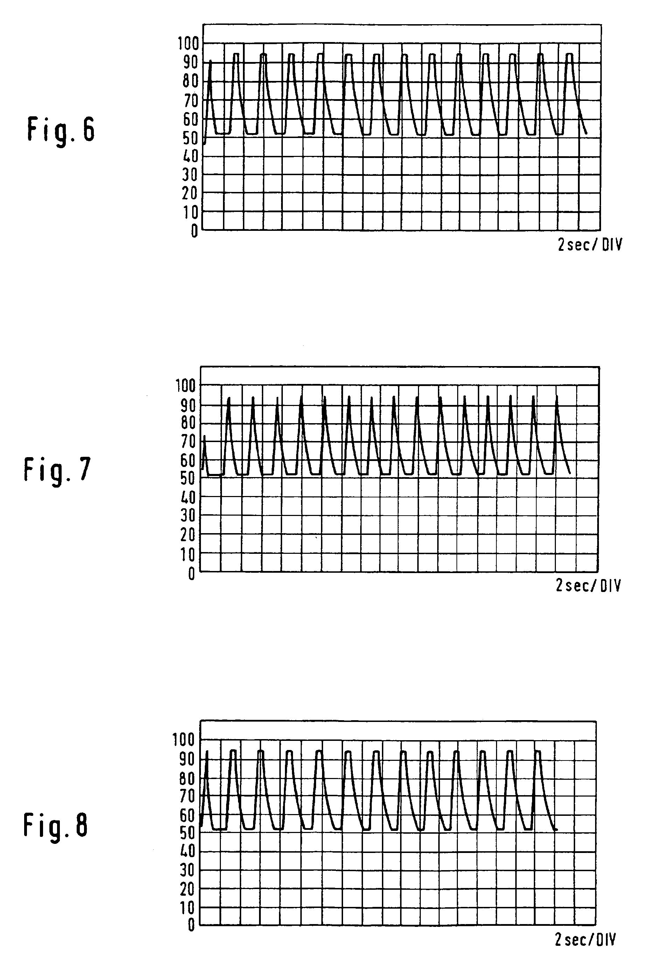

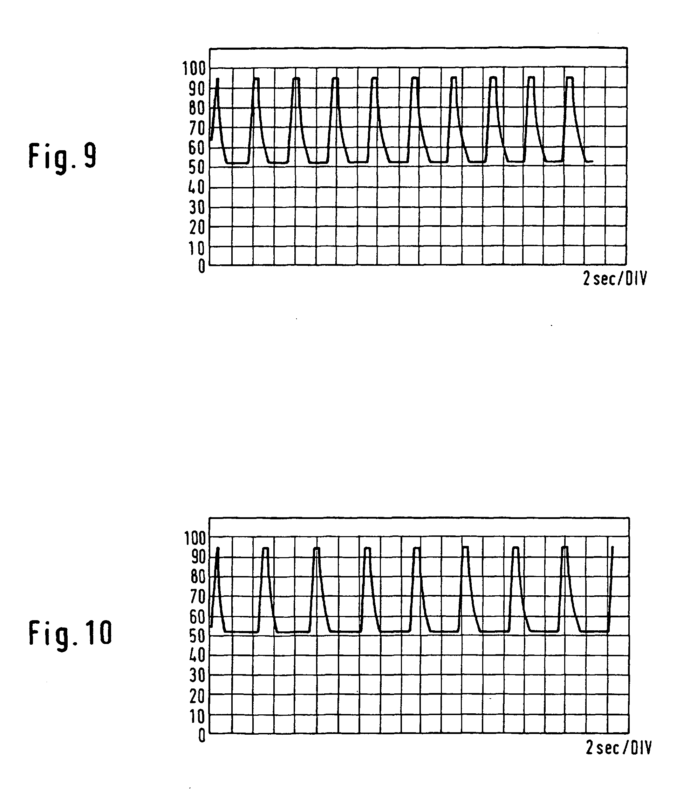

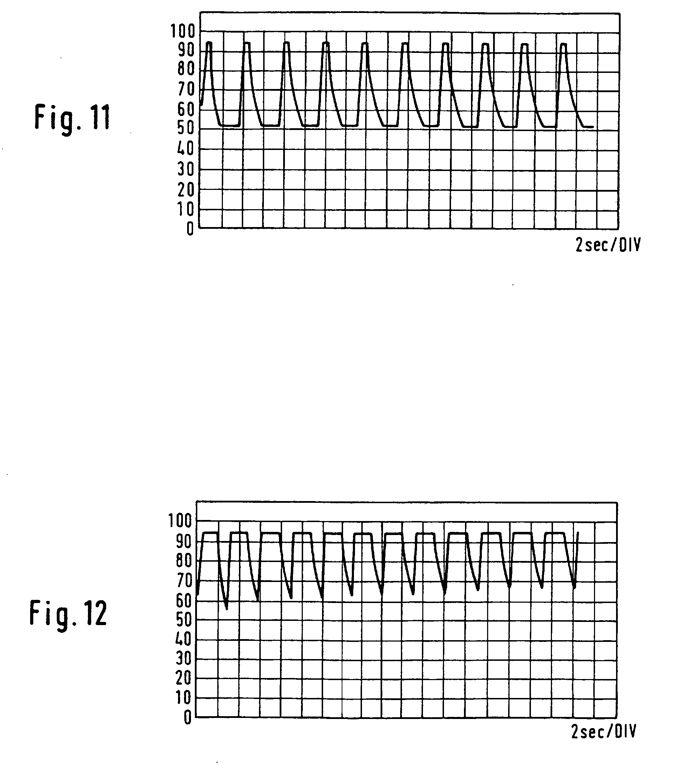

Ziel dieses Experiments ist die Ermittlung und Optimierung eines periodisch wechselnden Temperaturgradienten mit einem erfindungsgemäßen System. Hierzu wurde ein Reaktionstube aus Polypropylen, welches mit 300 µl autoklaviertem und bidestilliertem Wasser gefüllt war, in einen Metalltemperierblock, welcher über Peltierelemente gekühlt wurde, eingesetzt. In den Deckel wurde ein, kommerziell hältlicher Pt24-Chip integriert, der gleichzeitig als Heizelement und als Temperaturmeßfühler diente. Das Heizelement reicht bis in das Wasser hinein. In einem benachbarten Reaktionstube wurde die Einstellung und Beibehaltung der Standtemperatur (Temperatur des Temperierblocks) mit einem BBC-Temperaturmeßgerät M 4011 überwacht. In Figur 5 ist der Versuchsaufbau schematisch wiedergegeben. Dieser Aufbau wurde unter Anwendung unterschiedlicher Temperaturintervalle betrieben. Als Einzeit wird die Zeitdauer bezeichnet, in der die Heizung aktiv ist und als Auszeit wird das Zeitintervall zwischen den Heizzyklen bezeichnet. In den Figuren 6 bis 12 sind die Ergebnisse der Versuche angegeben. Es ist erkennbar, daß die Durchführung gemäß Figur 12 wohl keine sinnvolle Temperaturwechselbehandlung möglich macht, da die Zeiten für eine Rehybridisierung der Nukleinsäuren vermutlich ausreichend sind. Anhand dieser Versuche kann ein Fachmann für sein spezielles System (spezielle Geometrien, Heizleistungen etc.) die für seine Bestimmung besten Bedingungen ermitteln.

**Tabelle 1**

| **Standtemp./°C** | **gem. Temp./°C** | **Einzeit / ms** | **Auszeit /ms** | **Anhang-Nr.** |
|---|---|---|---|---|
| 4 | 4,9 | 800 | 2000 | FIG 6 |
| 10 | 11,0 | 400 | 2000 | FIG 7 |
| 10 | 10,6 | 800 | 2000 | FIG 8 |
| 10 | 11,0 | 800 | 3000 | FIG 9 |
| 10 | 10,9 | 800 | 4000 | FIG 10 |
| 20 | 20,2 | 800 | 3000 | FIG 11 |
| 20 | 20, 5 | 2000 | 1000 | FIG 12 |

## Patentansprüche

1. System zur Temperaturwechselbehandlung von nukleinsäurehaltigen Flüssigkeiten in einem Gefäß (7), wobei das System ein wiederverwertbares Temperierelement (10) , ein Gefäß (7) und ein disposibles Heizelement (5) enthält, wobei das Heizelement (5) integrierter Bestandteil des Gefäßes (7) oder eines Deckels (1) des Gefäßes ist und zur Durchführung der Behandlung in die Flüssigkeit hineintaucht.

2. Verwendung eines Systems gemäß Anspruch 1 zur Behandlung von Nukleinsäuren in einer Flüssigkeit unter Einstellung von zwei oder mehr Temperaturen mittels des Kühl- und des Heizelements.

3. Verwendung gemäß Anspruch 2, **dadurch gekennzeichnet, daß** die disposible Vorrichtung der Deckel eines Gefäßes ist.

4. Verwendung gemäß Anspruch 2 oder 3, **dadurch gekennzeichnet, daß** das Heizelement während des Heizvorganges in die Flüssigkeit hineinragt.

5. Verfahren zum Nachweis einer Nukleinsäure einer Probe durch
a) Freisetzen der Nukleinsäure, auf der der Nachweis beruhen soll, aus Kompartimenten, in denen sie enthalten ist, in einem Gefäß in einem System gemäß Anspruch 1,
b) Vermehrung von Sequenzinformationen, die auf der Anwesenheit der Nukleinsäure in dem Gefäß beruht und
c) Nachweis der Sequenzinformation.

6. Verfahren gemäß Anspruch 5, **dadurch gekennzeichnet, daß** die Vermehrung der Sequenzinformation in Temperaturzyklen durchgeführt wird.

7. Verfahren gemäß Anspruch 6, **dadurch gekennzeichnet, daß** die Temperaturzyklen mit Hilfe eines Heiz- und eines Kühlelementes vorgenommen werden, wobei das Heizelement Teil einer disposiblen Vorrichtung ist.

## Claims

1. System for subjecting liquids containing nucleic acids to temperature changes in a vessel (7) comprising a reusable temperature control element (10), a vessel (7) and a disposable heating element (5), wherein the heating element (5) is an integral component of the vessel (7) or of a vessel lid (1) and dips into the liquid to carry out the treatment.

2. Use of a system according to claim 1 to treat nucleic acids in a liquid at two or more temperatures that are set by means of the cooling and heating element.

3. Use according to claim 2, **characterized in that** the disposable device is the lid of a vessel.

4. Use according to claim 2 or 3, **characterized in that** the heating element extends into the liquid during the heating process.

5. Method for detecting a nucleic acid in a sample by
a) releasing the nucleic acid which is to be the basis for the detection from compartments in which it is contained, in a vessel in a system according to claim 1,
b) replicating sequence information which is based on the presence of the nucleic acid in the vessel and
c) determination of the sequence information.

6. Method according to claim 5, **characterized in that** the sequence information is replicated in temperature cycles.

7. Method according to claim 6, **characterized in that** the temperature cycles are formed with the aid of a heating and cooling element where the heating element is part of a disposable device.

## Revendications

1. Système pour le traitement par cyclage thermique de liquides contenant des acides nucléiques dans un récipient (7), le système contenant un élément de thermostatisation recyclable (10), un récipient (7) et un élément de chauffage à jeter (5), l'élément de chauffage (5) représentant un constituant faisant partie intégrante du récipient (7) ou d'un couvercle (1) du récipient et plongeant dans le liquide pour la mise en oeuvre du traitement.

2. Utilisation d'un système selon la revendication 1, pour le traitement d'acides nucléiques dans un liquide en réglant deux températures ou plus au moyen de l'élément de refroidissement et de l'élément de chauffage.

3. Utilisation selon la revendication 2, **caractérisée en ce que** le dispositif à jeter est le couvercle d'un récipient.

4. Utilisation selon la revendication 2 ou 3, **caractérisée en ce que** l'élément de chauffage pénètre dans le liquide, au cours du processus de chauffage.

5. Procédé pour le décèlement d'un acide nucléique d'un échantillon par :
a) libération de l'acide nucléique, sur lequel doit se baser le décèlement, à partir de compartiments dans lesquels il est contenu, dans un récipient dans un système selon la revendication 1 ;
b) multiplication des informations de séquences, qui s'appuie sur la présence de l'acide nucléique dans le récipient ; et
c) décèlement des informations de séquences.

6. Procédé selon la revendication 5, **caractérisé en ce qu'**on met en oeuvre la multiplication des informations de séquences dans des cycles de température.

7. Procédé selon la revendication 6, **caractérisé en ce qu'**on procède aux cycles de température à l'aide d'un élément de chauffage et d'un élément de refroidissement, l'élément de chauffage faisant partie d'un dispositif à jeter.


### SEQUENZPROTOKOLL

(1) ALLGEMEINE ANGABEN:
   **(i) ANMELDER:**
      (A) NAME: Boehringer Mannheim GmbH
      (B) STRASSE: Sandhoferstr. 116
      (C) ORT: Mannheim
      (E) LAND: DE
      (F) POSTLEITZAHL: 68305
      (G) TELEFON: 0621 759 4348
      (H) TELEFAX: 0621 759 4457
   (ii) BEZEICHNUNG DER ERFINDUNG: System zur Temperaturwechselbehandlung von Probenfluessigkeiten
   (iii) ANZAHL DER SEQUENZEN: 2
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LÄNGE: 20 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Otigodesoxyribonukleotid"
   (iii) HYPOTHETISCH: NEIN
   (xi) SEQUENZBESCHREIBUNG: SEQ ID NO: 1:
      AGACAGTACA GCCAGCCTCA 20
(2) ANGABEN ZU SEQ ID NO: 2:
   (i) SEQUENZKENNZEICHEN:
      (A) LÄNGE: 21 Basenpaare
      (B) ART: Nucleotid
      (C) STRANGFORM: Einzelstrang
      (D) TOPOLOGIE: linear
   (ii) ART DES MOLEKÜLS: Sonstige Nucleinsäure
      (A) BESCHREIBUNG: /desc = "Oligodesoxyribonukleotid"
   (iii) HYPOTHETISCH: NEIN
   (xi) SEQUENZBESCHREIBUNG: SEQ ID NO: 2:
      GACTTCAAAT TTCTGCTCCT C 21